# EUROPÄISCHE PATENTANMELDUNG

(11) **EP 0 882 474 A2**
(43) Veröffentlichungstag der Anmeldung: **09.12.1998**
(21) Anmeldenummer: 98810474.1
(22) Anmeldetag: 20.05.1998
(51) Int. Cl.: A63B 24/00, A63B 5/00

(54) **Tragbare Sprungkraft-Messplattform**

(30) Priorität: 04.06.1997 CH 1331/97
(71) Anmelder: K.K. Holding AG, 8408 Winterthur (CH)
(72) Erfinder: Stüssi, Edgar, Prof. Dr., 3095 Spiegel (CH)

(57) **Zusammenfassung**

Beim Training für viele Sportarten spielt die Sprungkraft der Beinmuskulatur eine massgebende Rolle. Messtechnisch sollten daher vor allem die Absprungkraft bei Einzel- und Mehrfach-Absprüngen, aber auch die Aufsprung- oder Rückkehrkraft sowie die zwischen beiden Messpunkten liegende Flugzeit erfasst werden.

Erfindungsgemäss wird dies erreicht durch drei in Dreieckform angeordnete, fixierte sowie mit Kraftmess-Sensoren (6) bestückte Abstützeinheiten (2), die durch eine Grundplatte (3) untereinander derart stabilisiert sind, dass auch gleichzeitige Ab- und/oder Aufsprungkräfte für Einfach- und Mehrfachsprünge, unabhängig von der Bodenauflage (1), messbar sind.

## Beschreibung

Beim Training für viele Sportarten spielt die Sprungkraft der Beinmuskulatur eine massgebende Rolle. Messtechnisch sollten daher primär die Absprungkraft in Einzel- und Mehrfach-Absprüngen sowie sekundär die Aufsprung- oder Rückkehrkraft und die zwischen beiden Messpunkten liegende Flugzeit erfasst werden.

Zum Stand der Technik gehören Kontakt-Matten, welche durch eingebaute Flächenkontakte die Flugzeit messen, die lediglich gewisse Rückschlüsse auf die Absprungkraft zulässt, aber nicht deren exakten Werte wiedergibt. Der Vorteil dieser Matten liegt im Preis und in der einfachen Verlege- und Transportmöglichkeit. Hauptnachteil ist, dass die Absprungskraft, ihr zeitlicher Verlauf bei der Umsetzung in den Sprung und auch die Abfederung des Aufsprungs unbekannt bleiben. Dadurch sind wesentliche Merkmale der Trainingsresultate nicht erkennbar, was die Anwendung der Sprungmatte einschränkt.

Diese Nachteile sollen durch die erfindungsgemässe Sprungkraft-Messplattform behoben werden. Diese Plattform ist ebenfalls tragbar und lässt sich auf jedem Hartbelag aufstellen. Sie ist gekennzeichnet durch die Merkmale von Anspruch 1. Durch drei fixierte Abstützeinheiten ist sie statisch bestimmt, womit eine genaue Erfassung der auf sie wirkenden Kräfte möglich ist, weil Kraftmess-Sensoren in die Abstützeinheiten integriert sind. Erstmalig ist somit eine Messplattform gegeben, mit der die bei Sprüngen auftretenden Kräfte direkt in absoluten Werten gemessen werden können. Vor allem kann, beispielsweise bei Mehrfachsprüngen, in einer Sprungphase der vollständige Kraftverlauf vom Aufsprung bis zum Wiederabsprung erfasst und analysiert werden.

Für den trainierenden Sportler ist eine Dreieckform der Plattform selbst nicht sehr günstig. Im Rahmen der Erfindung kann diese deshalb durch eine weitere Abstützeinheit zu einem Viereck erweitert werden, wobei diese Abstützeinheit ebenfalls mit einem Kraftmess-Sensor bestückt sein kann. Es ist jedoch auch möglich, die Dreieckform durch Anlenkelemente zur angenäherten oder vollen Kreis- oder zu einer Polygonform zu ergänzen, ohne dass die mit der Dreieckform gemessenen Resultate verändert werden.

Grundplatte und Anlenkelemente sind dabei zusammensetz- oder zusammenlegbar, wodurch im zusammengelegten Zustand ein Paket mit reduzierten geometrischen Abmessungen entsteht. Am Einsatzort zur Kraftmessung wird das Paket aufgeklappt bzw. entsprechend zusammengesteckt, wodurch dann eine vergrösserte Messfläche zur Verfügung steht, die auch optisch durch den Springer ― mit dem Ziel, möglichst im Flächenschwerpunkt zu arbeiten ― leicht erfassbar ist.

Grundplatten und Anlenkelemente in Dreieckform sind dabei mit Vorteil derart miteinander verbunden, dass die Schwenkachse möglichst nahe bei der Verbindungslinie von zwei benachbarten Kraftmess-Sensoren liegt. Diese sollten derart angeordnet sein, dass sie sich in unmittelbarer Nähe einer Schwenkachse befinden, wobei in unmittelbarer Nähe einer Schwenkachse nur zwei Kraftmess-Sensoren notwendig sind.

Durch die Verwendung von dreieckigen Bauelementen und einer Anordnung der Abstützeinheiten in den Eckbereichen der Dreiecke oder deren unmittelbarer Umgebung ist eine stabile, nicht kippelige Plattenauflage gegeben.

Mit Vorteil werden Anlenkelemente, die bevorzugt nur eine Abstützeinheit haben, mit einer Grundplatte derartig verbunden, dass ihre Abstützeinheit nicht in der Nähe der Grundplatte liegt

Mit der Erfindung ergibt sich somit eine messtechnisch einfache und genaue Anordnung, mit einer mindestens verdoppelten Grösse der Sprungfläche, ohne jede Unfallgefahr sowie mit leichter Transportmöglichkeit. Die Erfindung ermöglicht weiterhin die Verbindung der wichtigsten Parameter in einer Konstruktionsform:
- Genaue Erfassung der Sprungdaten, insbesondere des Kraftverlaufs von Aufsprung und Wiederabsprung bei Mehrfachsprüngen.
- Optisch einfache Erfassung der Plattform durch die Trainierenden, ohne Unfallgefahr.
- Einfache Transport- und Aufstellmöglichkeit.
- Preisgünstige und betriebssichere Form der Plattform.

Die Gedanken der Erfindung sollen anhand der Figuren näher erklärt werden:
Es zeigen:
- Fig. 1: Schrägansicht der erfindungsgemässen Plattform mit angelenkten Elementen gestrichelt.
- Fig. 2: Schnitt durch Plattform, entlang Linie A-A von Fig. 1;
- Fig. 3: Schnitt durch Plattform, entlang Linie B-B von Fig. 1, mit aufklappbarem Anlenkelement.
- Fig. 4: In gleicher Darstellung wie Fig. 3 eine weitere Ausführungsform mit einhängbarem Anlenkelement;
- Fig. 5: eine Aufsicht auf verschiedene Ausführungensmöglichkeiten der neuen Messplattform, die alle übereinander dargestellt sind,
- Fig. 6: mehrere Messplattformen mit Grundplatten in der Form von gleichseitigen Dreiecken, zu einer Absprung/Aufsprung-Messstrecke vereinigt,
- Fig. 7: eine Detaildarstellung einer einstellbaren Abstützeinheit im Schnitt A-A von Fig. 1,
- Fig. 8: einen flächenhaften, polygonartigen Aufbau aus einer Vielzahl von Dreieckelementen,
- Fig. 9: einen Querschnitt entlang der Linie IX-IX von Fig. 5 zur Darstellung einer Verbindung zwischen einer Grundplatte und einem Anlenkelement der erfindungsgemässen Messplattform, und
- Fig. 10: eine Variante zu der in Figur 9 dargestellten Verbindung zwischen Grundplatte und Anlenkelementen.

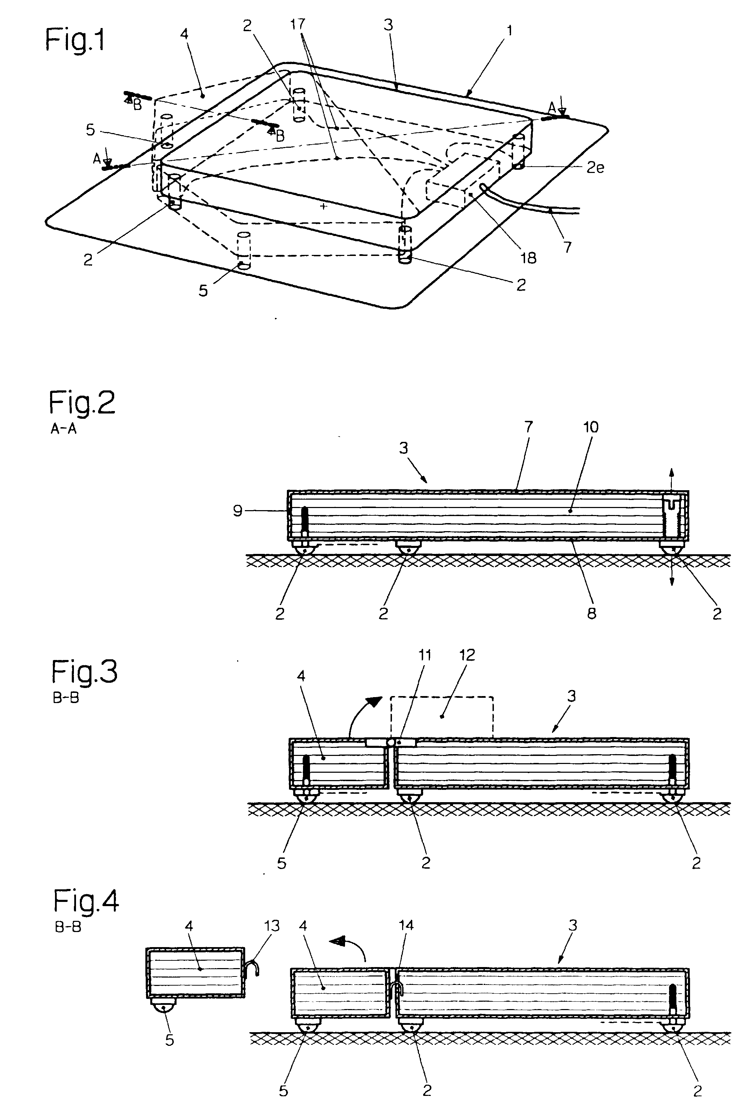

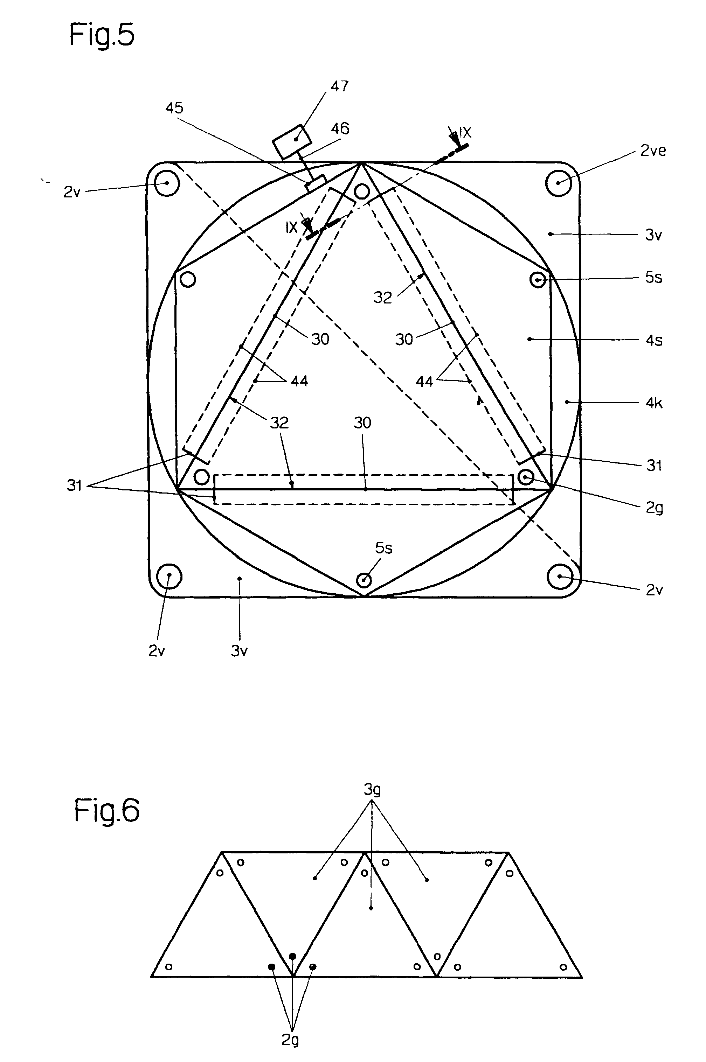

Auf einem möglichst harten Boden 1 (Fig. 1) stützt sich die Messplattform in der Grundausführung über drei in Form eines Dreiecks angeordnete Abstützeinheiten 2 ab. Diese sind durch eine Grundplatte 3 miteinander verbunden und untereinander stabilisiert, wobei die Anordnung der Abstützeinheiten 2 an sich ein beliebiges Dreieck bilden kann, vorzugsweise jedoch ein gleichseitiges oder ein rechtwinkliges Dreieck ergibt, wie in Fig. 5 verdeutlicht.

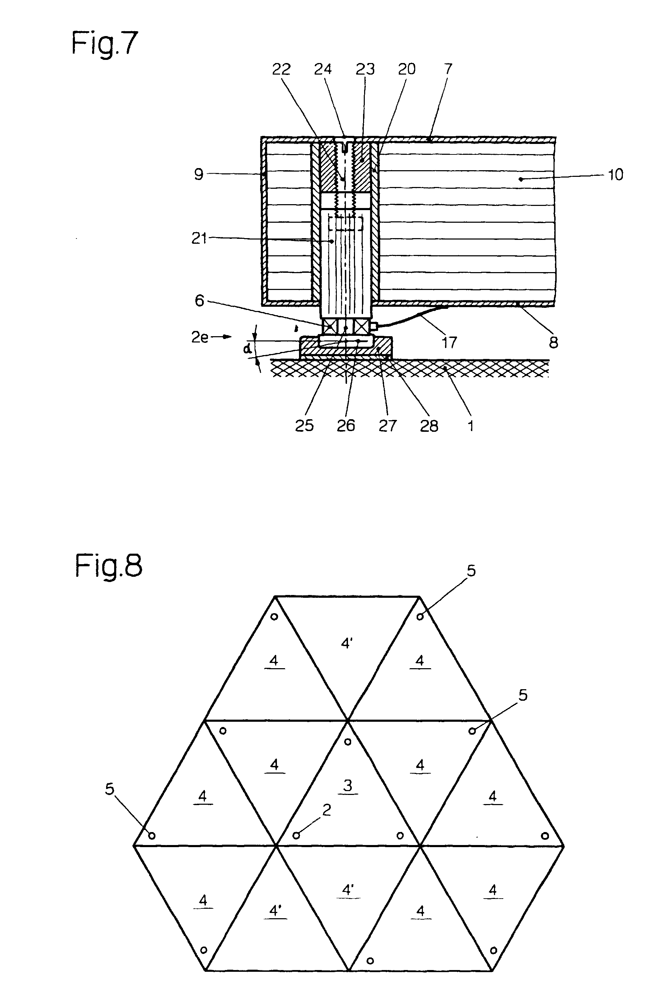

In Fig. 1 ist die in ausgezogenen Linien dargestellt Grundplatte 3 zu einem Viereck - die Dreiecks-Grundform ist durch eine punktierte Linie angedeutet - erweitert und über eine zusätzliche Abstützeinheit 2e, die in ihrer Höhe einstellbar ausgebildet sein kann, auf dem Boden 1 abgestützt. Die Abstützeinheiten 2, 2e der Grundplatte 3 sind generell mit Kraftmess-Sensoren 6 (Fig. 7) bestückt, wobei das jeweilige Messsignal über Kabel 17 zu einem Sammelpunkt 18, der, mit Verstärker versehen, in die Grundplatte 3 integriert ist, geführt und zusammengeschaltet oder als Einzelsignal nach aussen zur Datenverarbeitung geleitet wird. Diese Datenverarbeitung erfolgt nach bekannten Methoden und soll hier nicht weiter behandelt werden, weil sich die Erfindung auf die Plattenanordnung konzentriert.

Als Variante zu der Viereck-"Erweiterung" sind in Fig. 1 an Dreieckseiten der Grundplatte 3 ansetzbare Anlenkelemente 4 in gestrichelter Darstellung eingezeichnet, die ebenfalls als Erweiterung der dreieckigen Grundplatte 3 Verwendung finden. Sie werden, wie in Fig. 5 gezeigt, vorzugsweise in Verbindung mit einer gleichseitigen Dreiecks-Grundform verwendet und sind selbst in ihre Grundfläche beispielsweise als stumpfwinklige Dreiecke oder als Kreissegmente (Fig. 5) ausgebildet. Ihre Abstützung auf dem Boden 1 erfolgt über Abstützeinheiten 5, die in ihrem von der Grundplatte 3 entfernten Bereich liegen und meist nicht mit Sensorelementen 6 versehen sind.

Vor allem für die Grundplatte 3 wird eine möglichst grosse Steifigkeit gefordert, damit ihre Eigenfrequenz deutlich über 100 Hz liegt. Bekannterweise wird ein Optimum von minimalem Gewicht und maximaler Steifigkeit mit Wabenkonstruktionen erreicht, wie sie in der Raumfahrt bekannt geworden sind. Preiswerte Annäherungen sind Leichtmetallplatten mit leichten Füllmaterialien als Zwischenschichten, z.B. in Sandwich-Bauweise. Beispielsweise sind eine Oberplatte 7 (Fig. 2), eine Unterplatte 8 und eine Umfassungswand 9 zu einem Hohlkörper aus einer Al-Legierung vereinigt, der mit fest eingepressten oder eingegossenen Füllmaterialien 10 ausgestopft ist. Geeignet sind beispielsweise Holzplatten oder Kunststoffe. Die Anlenkelemente 4 können in der gleichen Weise aus einer Blechhülle bestehen, die mit Füllmaterialien gefüllt ist.

Fig. 3 zeigt eine als gleichseitiges Dreieck ausgeführte Grundplatte 3, an die ein Anlenkelement 4 angelenkt ist, das mittels Scharnier 11 an der Grundplatte 3 montiert ist. Damit kann es zum Transport in die Pos. 12 umgeklappt werden, was voraussetzt, dass das Anlenkelement 4 höchstens 1/3 der Grundplatte 3 entspricht, also als stumpfwinkliges, gleichschenkliges Dreieck ausgebildet ist.

In Fig. 4 ist ein Anlenkelement 4, das in eine Koppelöffnung 14 eingehängt wird, mittels eines Koppelelements 13 an die wiederum gleichseitige Dreiecks-Grundplatte 3 angekoppelt. Diese Variante erlaubt eine einfache Demontage der Anlenkelemente 4 zum Transport. Gleichzeitig ist ihre Formgebung freier, so dass sie als Kreissegment ausgebildet sein können.

In Fig. 5 sind nochmals die vorstehend beschriebenen, relativ einfachen Beispiele verschiedener Ausführungsformen der neuen Messplattform zusammengefasst, wobei alle drei Varianten mit einem gemeinsamen Zentrum ineinander dargestellt sind.

Eine Grundplatte 3v, die - wie durch eine punktiert eingezeichnete Hypotenuse angedeutet, die Form eines rechtwinkligen Dreiecks hat und die Abstützeinheiten 2v miteinander verbindet, ist zu der Viereckplattform mit der zusätzlichen Abstützeinheit 2ve erweitert. Eine Möglichkeit, dafür eine statisch stabile Auflage zu gewährleisten, besteht darin, diese zusätzliche Abstützeinheit 2ve in ihrer Höhe einstellbar auszubilden. Obwohl die Grundplatte 3v nur als eine einzige zusammenhängende Platte gezeigt ist, ist es selbstverständlich möglich, diese längs der punktierten Hypotenuse in zwei rechtwinklige Dreiecke zu unterteilen. Wie die Konstruktionen nach Fig. 3 und 4 können dabei die beiden Dreiecke durch Scharniere oder Koppelglieder zu der Viereckplattform vereinigt sein.

An die gleichseitige Dreiecks-Grundplatte 3g, die sich auf Abstützeinheiten 2g abstützt, sind längs aller drei Seiten entweder stumpfwinklige Anlenkelemente 4s oder kreissegmentförmige Anlenkelemente 4k angesetzt, beide abgestützt auf je einer Abstützeinheit 5s, die sensorbestückt oder nicht mit einem Sensor 6 versehen sein kann.

Selbstverständlich können für die Grundplatte 3 und/oder die Anlenkelemente 4 auch andere Dreiecksformen, z.B. schiefwinklige, sowie vier- oder mehreckige Grundformen verwendet werden. Die gezeigten Dreiecksformen erlauben jedoch auf besonders einfache Weise eine geschlossene Messfläche zu erreichen.

Fünf gleichseitige Grundplatten 3g sind in Fig. 6 zu einer Mess-"Strasse" zusammengesetzt, mit der bei Mehrfachsprüngen, beispielsweise beim Dreisprung in der Leichtathletik, der erste Absprung, die erste Landung und der auf sie folgende erst Wiederabsprung, weiterhin eine zweite Landung und ein zweiter Wiederabsprung sowie unter Umständen eine dritte Landung erfasst werden können. Selbstverständlich ist ein derartiger Zusammenbau nicht auf eine einbahnige Strasse beschränkt, sondern kann auch andere Flächenformen annehmen.

So zeigt Fig. 8 den Aufbau einer polygonartigen Messfläche aus Grundelementen, die als gleichseitige Dreiecke ausgebildet sind. Neben mindestens einer Grundplatte 3, die sensorbestücke Abstützelemente 2 aufweist sind mit der Grundplatte 3 flächengleiche Anlenkelemente 4 vorgesehen; diese können eine, in diesem Fall mit Vorteil sensorbestückte, Abstützeinheit 5 haben oder ohne eine solche ausgebildet sein (Anlenkelemente 4'), so lange in unmittelbarer Nähe einer Verbindung zweier Elemente zwei sensorbestückte Abstützeinheiten 2 oder 5 vorhanden sind. Selbstverständlich ist es auch möglich eine Polygon-Messfläche nur aus Grundplatten 3 zusammenzusetzen.

Bei dem Beispiel einer konstruktiven Ausgestaltung einer Abstützeinheit 2 oder 5 nach Fig. 7 ist in die Blechumhüllung 7-9 einer Grundplatte 3 oder eines Anlenkelements 4 eine Hülse 20 eingeschweisst, in die ein Zylinder 21 eingeschoben ist. In dessen obere Stirnfläche greift eine, bei einer höheneinstellbaren Abstützeinheit 2e einstellbare, Verstellschraube 22 ein. Diese ist in einer, in der Hülse 20 fixierten, Gewindebuchse 23 geführt und durch eine Öffnung 24 in der Oberplatte 7 für eine Verstellung zugänglich.

Von unten ist in den Zylinder 21 eine weitere Schraube 25 mit einem flachen, scheibenförmigen Kopf 26 eingeschraubt, durch die der Sensor 6, gegebenenfalls unter Vorspannung gegen den Zylinder 21 gepresst wird. Ihr Kopf 26 ruht in einer Lagerschale 27, die ihrerseits über eine elastische Zwischenlage 28 auf dem Boden 1 aufliegt. Diese erlaubt infolge ihrer Zusammenpressbarkeit in gewissem Umfang "Höhenänderungen" und ermöglicht darüber hinaus Schwenkbewegungen um den Mittelpunkt des Schraubenkopfs 26 (Winkel a). Dadurch wird, in Verbindung mit oder anstatt der Höheneinstellbarkeit, eine statisch stabile Abstützung, beispielsweise bei einer Viereckplattform, erreicht.

Nicht höhenverstellbare Abstützeinheiten 2 oder 5 können ebenfalls in der vorstehend beschriebenen Konstruktion ausgeführt sein. Bei ihnen wird dann lediglich die Verstellschraube 22 unlösbar fixiert. Bei nicht sensorbestückten Abstützeinheiten 5 kann der Sensor 6 durch einen entsprechend dimensionierten Abstandshalter ersetzt sein.

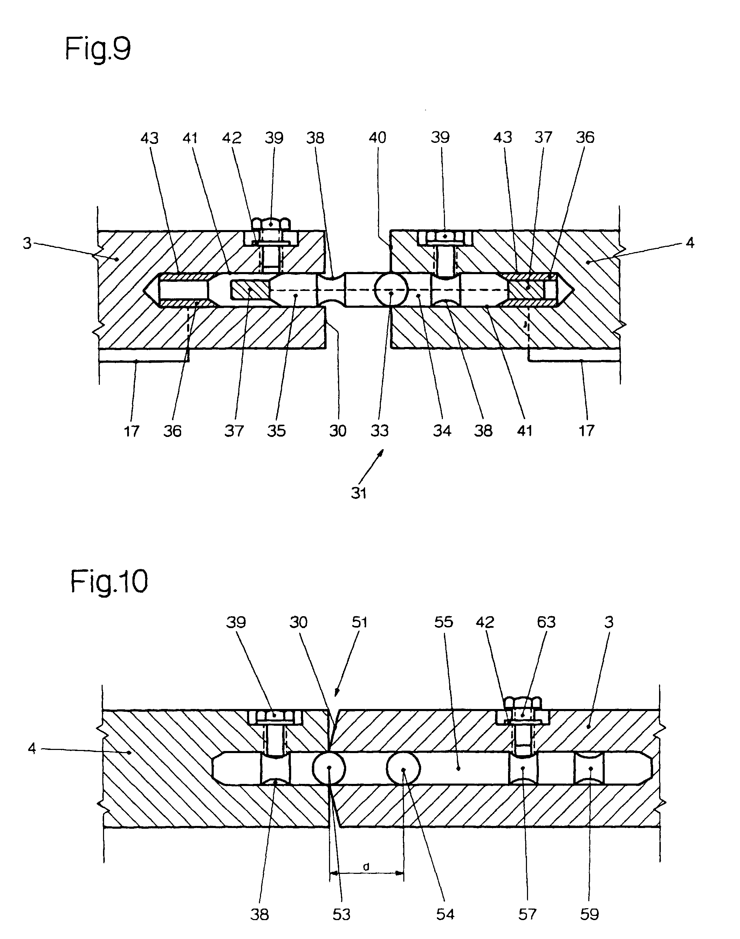

Eine Grundplatte 3 und die an ihren Rändern bzw. Stirnseiten 30 ( Fig. 5) schwenkbar angeordneten Anlenkelemente 4 sind über in einem Querschnitt in Fig. 9 dargestellte Gelenkelemente 31 miteinander verbunden. Pro Schwenkachse 32 (Fig. 5) sind jeweils zwei Gelenkelemente 31 vorhanden. Die Gelenkelemente 31 sind jeweils in den Eckbereichen der Grundplatte 3 sowie der Anlenkelemente 4 angeordnet. Der Abstand von den Ecken der Grundplatte 3 sowie von den Ecken der Anlenkelementen 4 ist lediglich durch die benötigte Materialdicke für das Einsetzen der Gelenkelemente 31 gegeben. Die Lage der Gelenkelemente 31 ist in Fig. 5 durch Striche angedeutet.

Die Gelenkelemente 31, wie sie in Fig. 9 dargestellt sind, haben ein Scharniergelenk 33, welches an beiden Seiten je einen Zapfen 34 bzw. 35 aufweist. Der Zapfen 35 ist nicht vollständig eingesteckt dargestellt, um die beiden Steckerteile 36 und 37 der unten aufgeführten schwimmenden Steckverbindung schematisch andeuten zu können. Die Zapfen 34 und 35 haben einen runden oder einen eckigen Querschnitt und an ihrem freien Endbereich eine umlaufende Nut 38. In jede Nut 38 greift von der Oberfläche der Grundplatte 3 bzw. eines Anlenkelements 4 her eine Feststellschraube 39, mit der jeder Zapfen gegen Verdrehen sicherbar ist. In den Stirnseiten 30 sowie 40 sind zu den Zapfen 34 und 35 passende Sacklöcher 41 vorhanden. Die Schraubenschaftlänge ist derart gewählt, dass bei eingeschraubter Schraube 39 die Oberseite des Schraubenkopfs bündig mit der jeweiligen Oberfläche verläuft, damit beim beispielsweisen Sporteinsatz kein Sportler am Schraubenkopf hängen bleiben oder über ihn stolpern kann. Gegen selbständiges Herausdrehen ist unter jedem Schraubenkopf eine Federscheibe 42 angeordnet.

Die im Querschnitt kreisförmige Ausgestaltung der Zapfen 34 und 35 wurde nur wegen der besseren Herstellbarkeit gewählt. Die Verdrehsicherung erfolgt hier nur durch die Klemmkraft der Schrauben 39. Besser würde man jedoch einen verdrehfesten Formschluss zwischen den Zapfen und hierzu passenden Öffnungen anstelle der Sacklöcher verwenden. Es könnte beispielsweise ein dreieckiger, quadratischer oder rechteckiger Querschnitt mit einer entsprechenden Zapfenquerschnittssgestaltung gewählt werden. Zapfen und Öffnung würde man mit einer engen Spielpassung ausbilden. Im Gegensatz zum erwähnten Sackloch 41 mit kreisförmigem Querschnitt, welches durch eine einfache Bohrung herstellbar ist, müssten nicht-kreisförmige Öffnungen aufwendiger, beispielsweise mit einer Räumnadel, hergestellt werden. Es könnten nicht-kreisförmige Sacklöcher auch durch Fräsen hergestellt werden, wenn die Fräsnut anschliessend mit einer Abdeckplatte abgedeckt wird. Eine Verdrehung der Zapfen in den Öffnungen derart, dass die Achse des Scharniergelenks 33 nicht mehr parallel zur Schnittgeraden paralleler Ebenen zu den Oberflächen der Grundplatte 3 und dem entsprechenden Anlenkelement 4 verläuft, würde nämlich eine Kraftaufnahme im entsprechenden Scharniergelenk 33 bewirken, wodurch dann das unten beschriebenen Messresultat verfälscht wäre.

Im Endbereich jedes Sacklochs 41 ist ein schwimmend gelagerter erster Steckerteil 36 angeordnet. Ein hierzu passender zweiter Steckerteil 37 ist an jeder Stirnseite der Zapfen 34 und 35 angeordnet. Für die schwimmende Lagerung können unterschiedliche Konstruktionen gewählt werden. Man kann beispielsweise die Stecker 36 sowie 37 in ein Weichgummibett einbetten und einen der Steckerteile, hier beispielsweise den Steckerteil 36, mit einem Einlauftrichter 43 versehen. Sämtliche Kraftmess-Sensoren 6 sind über ihre Energieversorgungs- und Signalkabel 17 mit sämtlichen Steckerteilen 35 verbunden; d.h. sämtliche Steckerteile 36 einer Einheit sind energetisch und signalmässig miteinander über in Fig. 5 gestrichelt angedeutete Kabel 44 verschlauft. In eines der noch freien Sacklöcher wird ein mit einem Handgriff versehener Stecker 45, der einen zum Steckerteil 37 analogen Steckerteil aufweist, eingesteckt. Der Stecker 45 ist dann über ein Kabel 46 mit einer Energieversorgungs- und Auswerteelektronik 47 verbunden. Jeder der Kraftmess-Sensoren 6 hat einen elektronischen Code, an dem die Elektronik 47 jeden Kraftmess-Sensor 6 erkennen und seine Messwerte zur Verarbeitung abfragen kann. Aus der Höhe des Messsignals können, da die Lage der Sensoren 6 in jeder Einheit 3 bzw. 4 bekannt ist, die auf die Einheit wirkende Kraft, deren zeitlicher Verlauf sowie Ihr Einwirkungsort ermittelt werden.

Statt die erfindungsgemässe Messplattform zusammensteckbar aus Grundplatte und Anlenkelementen auszubilden, kann sie derart aufgebaut werden, dass die Anlenkelemente 4 auf die Oberfläche der Grundplatte 3 einklappbar sind. Um ein Ein- und Ausklappen der Anlenkelemente 4 zu erreichen, wird beispielsweise das Gelenkelement 31 in das in Fig. 10 gezeigte abgeändert. Das in Fig. 10 dargestellte Gelenkelement 51 hat, im Gegensatz zu dem in Fig. 9 dargestellten, zwei Scharniergelenke 53 und 54. Der beispielsweise in die Grundplatte 3 einsteckbare Zapfen 55 hat, im Gegensatz zum Zapfen 35, zwei voneinander distanzierte, umlaufende Nuten 57 und 59. Der andere Zapfen 61 ist analog zum Zapfen 34 ausgebildet. Die Steckerteile für die Energieversorgung und die Signalübertragung sind analog zu denjenigen des Gelenkelements 31 ausgebildet und hier nicht dargestellt. Sollen die Anlenkelemente über die Grundplatte geklappt werden, so werden die Schrauben 63 in der Grundplatte 3 gelöst und der Zapfen 55 herausgezogen, bis das Scharniergelenk 54 am Ort des in Fig. 10 gezeichneten Scharniergelenks 53 liegt. Das Scharniergelenk 53 liegt dann vor der Stirnseite 30 der Grundplatte 3 in einem Abstand d, der um eine Toleranz grösser ist als der doppelte Abstand der Zapfenachse von den Oberflächen n von Grundplatte und Anlenkelement. Genaugenommen müsste der Abstand d die Summe bestehend aus dem Abstand der Zapfenachse des Zapfens 61 von der Oberfläche des Anlenkelements 4 plus dem Abstand der Zapfenachse des Zapfens 55 von der Oberfläche der Grundplatte 3 sein. Da aber die beiden Oberflächen in der Regel ineinander übergehen sollen, kann die oben aufgeführte Vereinfachung verwendet werden.

Referenzliste
- 1: Bodenauflage
- 2,2g,2s,2v,2ve: Abstützeinheiten
- 3,3g,3v: Grundplatte
- 4,4k,4s: Anlenkelement
- 5, 5s: Abstützeinheiten
- 6: Kraftmess-Sensor
- 7: Oberplatte
- 8: Unterplatte
- 9: Umfassungswand
- 10: Füllmaterial
- 11: Scharnier
- 12: Umgeklappte Position
- 13: Koppelelement
- 14: Koppelöffnung

- 17: Kabel
- 18: Sammelpunkt

- 20: Hülse
- 21: Zylinder
- 22: Verstellschraube
- 23: Gewindebüchse
- 24: Öffnung
- 25: Schraube
- 26: scheibenförmiger Schraubenkopf
- 27: Lagerschale
- 28: Zwischenlage

- 30: Stirnseite der Grundplatte
- 31: Gelenkelement
- 32: Schwenkachse
- 33: Scharniergelenk
- 34,35: Zapfen
- 36,37: Steckerteile
- 38: Nut
- 39: Schraube
- 40: Stirnseite eines Anlenkelements
- 41: Sackloch
- 42: Federscheibe
- 43: Einlauftrichter
- 44: Kabel
- 45: Stecker
- 46: Kabel
- 47: Energieversorgungs- und Auswerteelektronik

- 51: Gelenkelement
- 53, 54: Scharniergelenk
- 55: Zapfen
- 57,59: Nut

- 61: Zapfen
- 63: Schraube

## Patentansprüche

1. Tragbare Sprungkraft-Messplattform für Sporttrainingszwecke gekennzeichnet durch drei in Dreieckform angeordnete, fixierte sowie mit Kraftmess-Sensoren (6) bestückte Abstützeinheiten (2), die durch eine Grundplatte (3) untereinander derart stabilisiert sind, dass auch gleichzeitige Ab- und/oder Aufsprungkräfte für Einfach- und Mehrfachsprünge, unabhängig von der Bodenauflage, genau messbar sind.

2. Messplattform nach Anspruch 1, gekennzeichnet durch eine weitere gegebenenfalls einstellbare Abstützeinheit (2e), durch die die Dreieckform sowie die Grundplatte (3) zu einem Viereck erweitert werden.

3. Messplattform nach Anspruch 2, dadurch gekennzeichnet, dass die zusätzliche Abstützeinheit (2e) ebenfalls sensorbestückt ist.

4. Messplattform nach Ansprüchen 1, dadurch gekennzeichnet, dass die Grundplatte (3) mit Anlenkelementen (4) versehen ist, damit eine kreisähnliche Plattform entsteht, die an sich voll statisch stabil ist, ohne dass die Grundplatte (3) dadurch messtechnisch beeinflusst wird.

5. Messplattform nach Anspruch 4, dadurch gekennzeichnet, dass die Anlenkelemente (4) als stumpfwinklige Dreiecke ausgebildet und mittels Scharnieren (11) mit der Grundplatte (3) so verbunden sind, dass die stumpfwinkligen Dreiecke zum Transport umgeklappt werden können.

6. Messplattform nach Anspruch 4, dadurch gekennzeichnet, dass die Anlenkelemente (4) mittels Anlenkglied (13) und Koppelöffnung (14) mit der Grundplatte (3) so verbunden sind, dass sie zum Transport entfernt werden können.

7. Messplattform nach einem der Ansprüche 4 bis 6, dadurch gekennzeichnet, dass die Anlenkelemente (4) mit Abstützeinheiten (5) versehen sind, die nicht sensorbestückt und so dimensioniert sind, dass sie gleiche Bauhöhe aufweisen, wie die sensorbestückten Abstützeinheiten (2).

8. Messplattform nach Anspruch 7, dadurch gekennzeichnet, dass die Abstützeinheiten (5) der Anlenkelemente (4) ebenfalls sensorbestückt sind.

9. Messplattform nach einem der Ansprüche 1 bis 8, dadurch gekennzeichnet, dass die Abstützeinheiten (2 ,2e, 5) mittels elastischer Zwischenlagen (28) auf dem Boden (1) abgestützt sind.

10. Messplattform nach einem der Ansprüchen 1 bis 9, dadurch gekennzeichnet, dass die Grundplatte (3) in Leichtbauweise so gestaltet ist, dass sie im Minimum eine Eigenfrequenz von 100 Hz aufweist.

11. Messplattform nach einem der Ansprüche 1 bis 10, gekennzeichnet durch Gelenkelemente mit einem um eine Achse spielfrei schwenkbaren Gelenkkopf, an dem beidseits ein Einsteckflansch angeordnet ist sowie in jedem Eckbereich der Stirnseiten der Grundplatte sowie der zugeordneten Ankopplungsstirnseite jedes Anlenkelements angeordnete Einsteckhohlräume, in die die Einsteckflansche die Verbindung zwischen Grundplatte und betreffendem Anlenkelement bildend, einsteckbar sind.

12. Messplattform nach Anspruch 11, gekennzeichnet durch eine lösbare Klemmeinrichtung, welche jeden Einsteckflansch im jeweiligen Hohlraum gegen Herausrutschen sichert.

13. Messplattform nach einem der vorhergehenden Ansprüche, gekennzeichnet durch schwimmende, lösbare Stecker für die elektrische Energie- und Signalübertragung zwischen den einzelnen Kraftmess-Sensoren der Grundplatte und, gegebenenfalls, der Anlenkelemente.
